(19) Europäisches Patentamt
European Patent Office
Office européen des brevets

(11) EP 0 792 141 B1

(12) # EUROPEAN PATENT SPECIFICATION

(45) Date of publication and mention of the grant of the patent:
**25.09.2002 Bulletin 2002/39**

(21) Application number: **95937923.1**

(22) Date of filing: **15.11.1995**

(51) Int Cl.[7]: **A61K 7/16**

(86) International application number:
**PCT/GB95/02683**

(87) International publication number:
**WO 96/015767 (30.05.1996 Gazette 1996/25)**

(54) **ORAL TREATMENT**
ORALE BEHANDLUNG
TRAITEMENT BUCCAL

(84) Designated Contracting States:
**DE FR GB IT**

(30) Priority: **17.11.1994 GB 9423356**

(43) Date of publication of application:
**03.09.1997 Bulletin 1997/36**

(73) Proprietors:
- **UNILEVER PLC**
  **London EC4P 4BQ (GB)**
  Designated Contracting States:
  **GB**
- **UNILEVER N.V.**
  **3013 AL Rotterdam (NL)**
  Designated Contracting States:
  **DE FR IT**

(72) Inventors:
- **JOINER, Andrew**
  **Liverpool L17 7AR (GB)**
- **KHOSHDEL, Ezat**
  **Neston, Cheshire L64 9US (GB)**
- **POLYWKA, Robert**
  **Chester CH3 7HJ (GB)**

(74) Representative: **Newbould, Frazer Anthony et al**
**Unilever N.V.,**
**Patent Department,**
**Olivier van Noortlaan 120**
**3133 AT Vlaardingen (NL)**

(56) References cited:
**EP-A- 0 392 735**
**WO-A-92/07879**
**WO-A-94/26237**
**US-A- 5 039 813**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 792 141 B1

## Description

This invention relates to oral treatment, more particularly to compositions and methods for the treatment of teeth and other areas within the mouth. The invention relates more specifically to the treatment of surfaces within the mouth, especially the surfaces of the teeth but also other intra-oral surfaces such as the gums and mucosae, with one or more cosmetic, pharmaceutical or therapeutic agents.

Conventionally, active agents having cosmetic, pharmaceutical or therapeutic oral utility are delivered to the desired target areas in the mouth by deposition from oral compositions such as toothpastes, toothpowders, creams, mouthwashes or the like. Such actives include for example antiseptic agents, anticaries agents, anti-plaque agents, agents for alleviating problems of sensitive teeth, anti-stain agents and substances for treating bad breath.

Successful application of such conventional oral compositions relies essentially on two key factors: firstly, it is important that the active ingredient or ingredients which are to be delivered are provided in a form of composition which on the one hand is stable upon storage and maintains the essential active agent or agents in active form, and on the other hand allows the active(s) to be deposited from the composition onto the target surface(s) to impart the desired benefit or benefits to a sufficient degree and in an economical manner; secondly, it is essential that once deposited, the active or actives are retained on the target surface(s), so that when the treatment is completed and for example the mouth is rinsed to remove unwanted excess composition or residual components thereof, sufficient of the active substance(s) remains bonded to the target oral surface(s) for as long as possible so as to optimise the efficacy and economy of the treatment which it is desired to effect.

This bonding of active agent to the treated oral surfaces is generally of the nature of physisorption; that is to say, molecules of the active(s) are adsorbed onto the treated surface by virtue of physical intermolecular forces such as hydrogen bonding. Conventionally, it has been accepted that this form of retention of active substances on treated oral surfaces gives adequate results as regards achievable cosmetic/therapeutic benefits and economics. In the related art of cosmetic treatment of hair and skin, this is perhaps an explanation of why efforts over recent years have been towards improving the technology of deposition itself as a means for improving the efficacy and economy of many cosmetic products.

A major problem, however, associated with most known cosmetic products in general, even those which employ advanced patented deposition systems, is that once a substrate has been treated with an active agent, because of the very nature of the weak physical forces which bond the active to the substrate material, retention of the active agent at the desired site of application is frequently short-lived. In the context of oral treatment this is a major problem, because of the typical need to rinse the mouth following application of conventional oral compositions, e.g by brushing. Also, eating and drinking and the constant natural production of saliva in the mouth reduce even further the longevity of any cosmetic or therapeutic treatment applied to the teeth or other regions of the mouth. Hitherto the only way of improving things in this respect has been to repeat the oral treatment much more frequently, for example to brush the teeth after every meal or even more frequently than that.

Whilst in the field of cosmetic products for use on the hair or the skin there have in recent years been significant advances in deposition technology, there have been virtually no efforts made in a corresponding direction in the field of oral treatment. Thus, not only in this field are there serious shortcomings in the efficiency of deposition of oral cosmetic, pharmaceutical or therapeutic agents from known oral compositions, but this also represents a likewise unaddressed problem of uneconomical, non-environmentally friendly and wasted use of raw materials.

With the above criteria in mind, it was an object in the devising of the present invention to ameliorate the above-mentioned problems and to devise a means for achieving improved levels of retention of oral treatment agents on treated surfaces within the mouth, such that a desired one or more benefits wanted of a particular treatment can be achieved readily using conventional oral application and deposition technology but with a significantly longer lasting effect than has hitherto been possible to achieve using known means.

As relevant prior art to the present invention there may be mentioned EP-A-0392735 (Minnesota Mining and Manufacturing Company), which discloses certain azlactone-functional polymeric solid supports which are useful as complexing agents, catalysts, reagents, adsorbents, chromatographic supports and as biologically active supports for reaction with functional materials, one example of which is a protein, for example for use in protein synthesis. Whilst various polymer backbone species are disclosed for azlactone functionalisation in the formation of these polymeric supports, there is no disclosure or suggestion in the reference of the use of polymers which themselves may have a particular function which it may be desired to exploit. Azlactone-functional polymeric supports for covalently immobilizing biomolecules thereon are also disclosed in US 5200471 (Minnesota Mining and Manufacturing Company).

Similarly, US 5013795 (Minnesota Mining and Manufacturing Company) discloses substrates comprising a poly-alpha-olefin base polymer onto which has been grafted a 2-alkenyl azlactone moiety, which are disclosed for use in immunoassays and for immobilizing proteins.

It is also known, for example from US 4097444 (Hoechst AG) to use azlactone-based dyestuffs for dyeing water-insoluble thermoplastic polymers or polycondensates in mass, a result being that the dyed polymer exhibits,

inter alia, very good fastness properties with regard to thermosetting and light.

Various azlactone-containing copolymers are also known from US 4737560 (Minnesota Mining and Manufacturing Company) and US 4981933 (Polaroid Corporation), where it is disclosed that such azlactone copolymers can be used as intermediates for the ring-opening attachment (for various technical applications) of a variety of nucleophilic functional agents, such as dyes, catalysts, proteins, chelating agents, photographically useful compounds and therapeutically active agents.

EP-A-0435004 and EP-A-0437075 (both Dow Corning Corporation) describe, respectively, chlorobenzyl-functionalised and acrylic-functionalised siloxanes for use in the permanent waving of hair. Such functionalised siloxanes are useful as replacements for oxidising agents which are used in conventional perming treatments to cross-link reduced sulphur-sulphur bonds in hair proteins. Both of these disclosures, however, fail to address the problem of temporary retention on the hair of cosmetic agents such as film-forming conditioning and bodying/setting materials, and relate only to the well-documented use in the literature of certain types of cross-linking agent for the specific use in the cross-linking of a reduced hair protein structure.

What we have now surprisingly found is that in the context of oral treatment one can achieve good deposition and long-term retention of oral treatment agents on for example the teeth, gums and other interior surfaces of the mouth by use of particular molecular "handles" which serve to chemically bond the active agent(s) to characteristic functional groups exposed at the oral surface to be treated, whereby a stable, long-term union between surface and active agent is created which is substantially more resistant to the rigours of post-application treatments and influences than has hitherto been the case.

The molecular "handles" in question are azlactone groups, which are bonded chemically to molecules or groups which represent the cosmetically, pharmaceutically or therapeutically active oral species. The invention has as its basis the fact that the gums, mucosae and other intra-oral surfaces of the mouth present nucleophilic groups such as nitrogen-, oxygen- or sulphur-containing functional groups in the protein structure of which they are composed. Furthermore, normal healthy teeth always have associated with them a thin protein layer on their surface, known as the pellicle, which gives teeth a similar surface chemical nature to skin, so that they are equally applicable as oral substrate surfaces in the context of oral treatment of the kind with which the invention is concerned.

Accordingly, in a first aspect the present invention provides a method of cosmetic oral treatment wherein a cosmetic oral treatment agent is applied to an oral surface, the method comprising:

(a) providing the cosmetic oral treatment agent as molecules thereof having chemically bonded thereto at least one azlactone substituent moiety for reaction with a nucleophilic reactive site on the oral surface to be treated; and
(b) applying the azlactone-functionalised cosmetic oral treatment agent to the oral surface to effect reaction of the or each azlactone moiety with a or a respective nucleophilic reactive site on the surface;

whereby the molecules of cosmetic oral treatment agent are chemically bound to the surface to impart thereto one or more oral benefits characteristic of said oral treatment agent.

As used herein, the term "oral surface" is to be understood as meaning any surface inside the mouth, or even throat, which is susceptible to cosmetic, pharmaceutical or therapeutic treatment by one or more active species such as those disclosed and exemplified herein. Typically, such oral surfaces will be the teeth or gums, or possibly the mucosae or other areas of skin tissue within the mouth. However, in preferred contexts, the invention is applicable mainly to treatment of the teeth and gums, especially the teeth, the latter being those oral surfaces to which most preferred embodiments of the invention are directed.

The invention is applicable to oral surfaces as defined above by virtue of the fact that they constitute substrates which present at their surface at least one species of nucleophilic reactive site selected from nitrogen-containing functional groups, for example primary or secondary amine groups, oxygen-containing functional groups, for example hydroxyl groups, and sulphur-containing functional groups, for example thiol groups. Such functional groups may be an integral part of the tissue protein structure (e.g. in the case of the gums and mucosae) or may be present in a protein coating which is naturally present on the oral suface (e.g. in the case of pellicle on teeth).

Frequently, the oral surface may be characterised by exposed predominantly free amine groups, which provide particularly useful nucleophilic reactive sites for chemical bonding with the azlactone-functionalised molecules of oral treatment agent according to the invention, particularly when the reaction is carried out in an aqueous environment. Hydroxyl groups at the oral surface may become more important for reaction with the azlactone-functionalised oral treatment agent under non-hydrolytic conditions, e.g. in a non-aqueous, non-alcoholic environment or medium.

The azlactone-functionalised oral treatment agents according to the present invention provide active species which are both applicable to stabilised incorporation into oral compositions of conventional types, e.g. toothpastes, tooth powders, gels, creams, ointments, mouthwashes, etc., and are also able to be readily deposited from such compositions by conventional treatment regimes, such as brushing of the teeth, rinsing of the mouth, application of creams, ointments, lotions, pastes or powders, and the like. They may be formulated into a single formulation or they may be

formulated for multi-compartment containers into different formulations, e.g. one containing the azlactone-functionalised active agent and ingredients compatible therewith, and another comprising the remaining ingredients of the complete formulation.

The oral treatment active moiety of the azlactone-functionalised agents in accordance with the invention may be any oral benefit substance which is able to be functionalised by chemical reaction with the one or more azlactone substituent moieties and which imparts one or more cosmetic, pharmaceutical, therapeutic, preventative or other desirable benefits when applied to and reacted with an oral surface substrate in accordance with the invention.

In many embodiments, the oral treatment agent is a polymeric material which relies to at least some extent for its active properties on its polymeric structure, which is therefore preferred so that even when functionalised with the one or more azlactone substituent moieties the material retains its normal orally active function. Polymeric oral treatment agents will frequently be film-forming polymers. Such actives are particularly useful as anti-stain species, which prevent or hinder the deposit particularly on teeth of extrinsic dental stains such as those caused by smoking, beverages like tea and coffee, red wine, and antimicrobial substances (particularly cationic ones).

Other oral active species, which may or may not be polymeric, include other cosmetic, antiseptic, pharmaceutical or therapeutic agents, for example anti-caries agents, anti-plaque agents, agents for alleviating sensitive teeth, anti-calculus agents, anti-malodour agentts, teeth whitening agents, and pharmaceutically active substances for treating diseases or other medical conditions associated with the oral region.

Examples of suitable species which may constitute the oral treatment active moiety of the functionalised materials used in the present invention are given and described in further detail hereinbelow.

Methods for the preparation of azlactone-functionalised oral treatment agents for use in the present invention are described hereinbelow, as are methods by which the functionalised materials may be incorporated into oral compositions of the types already mentioned.

In a further aspect of the present invention, therefore, there is provided an oral composition in the form of a mouthwash, toothpaste, or tooth powder for treating an oral surface to impart thereto one or more cosmetic, pharmaceutical or therapeutic benefits, the composition comprising dissolved or dispersed particles of an oral treatment agent of which molecules thereof have chemically bonded thereto at least one azlactone substituent moiety capable of reaction with a nucleophilic reactive site on the oral surface to be treated.

In a further aspect, the present invention provides the use for treating an oral surface to impart thereto one or more cosmetic benefits of a cosmetic oral treatment agent, the molecules of which have chemically bonded thereto at least one azlactone substituent moiety capable of reaction with a nucleophilic reactive site on the oral surface to be treated.

In yet a further aspect, the present invention provides use of an azlactone-functionalised oral treatment agent for the manufacture of a medicament for use in the treatment of an oral condition by applying said oral treatment agent to an oral surface to be treated, wherein the oral treatment agent has chemically bonded thereto at least one azlactone substituent moiety capable of reaction with a nucleophilic reactive site on an oral surface to be treated.

Preferred embodiments of the present invention, and the various aspects and features thereof, will now be described in detail.

<u>Azlactone-functionalised oral treatment agent</u>

The oral treatment agent which characterises the various aspects of the present invention comprises at least one azlactone functionality chemically bonded to a base structural group, preferably a polymeric backbone, which, or a portion of which, delivers one or more desired oral treatment benefits when deposited on the target surface to be treated. By "azlactone" is meant the following group:

Formula A

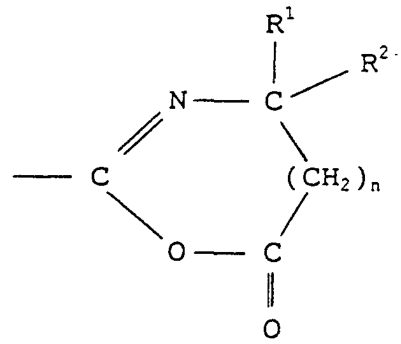

wherein $R^1$ and $R^2$ are the same or different and each is independently selected from H, an alkyl group having from 1 to 14 carbon atoms, particularly lower ($C_1$-$C_4$) alkyl e.g. methyl, a cycloalkyl group having from 3 to 14 carbon atoms, an aryl group having from 5 to 12 ring carbon atoms, an arenyl group having from 6 to 26 carbon atoms and from 0 to 3 S, N or 0 heteroatoms, or $R^1$ and $R^2$ taken together with the carbon atom to which they are joined can form a carbocyclic ring containing 4 to 12 ring atoms, and n is an integer from 0 to about 12, e.g. from 0 to about 3.

Although strictly speaking in the chemical art the name "azlactone" applies only to that group of formula A above in which n=0, as used herein the term "azlactone" is to be understood as applying to all such groups defined in Formula A above, for all values of n.

When the azlactone-functionalised active agent is applied to an oral surface substrate in accordance with the invention, namely a surface having exposed thereat at least one nucleophilic group such as amine, hydroxyl or thiol, the azlactone group reacts with the nucleophile, resulting in a ring-opening reaction represented by the following equation:

Equation 1

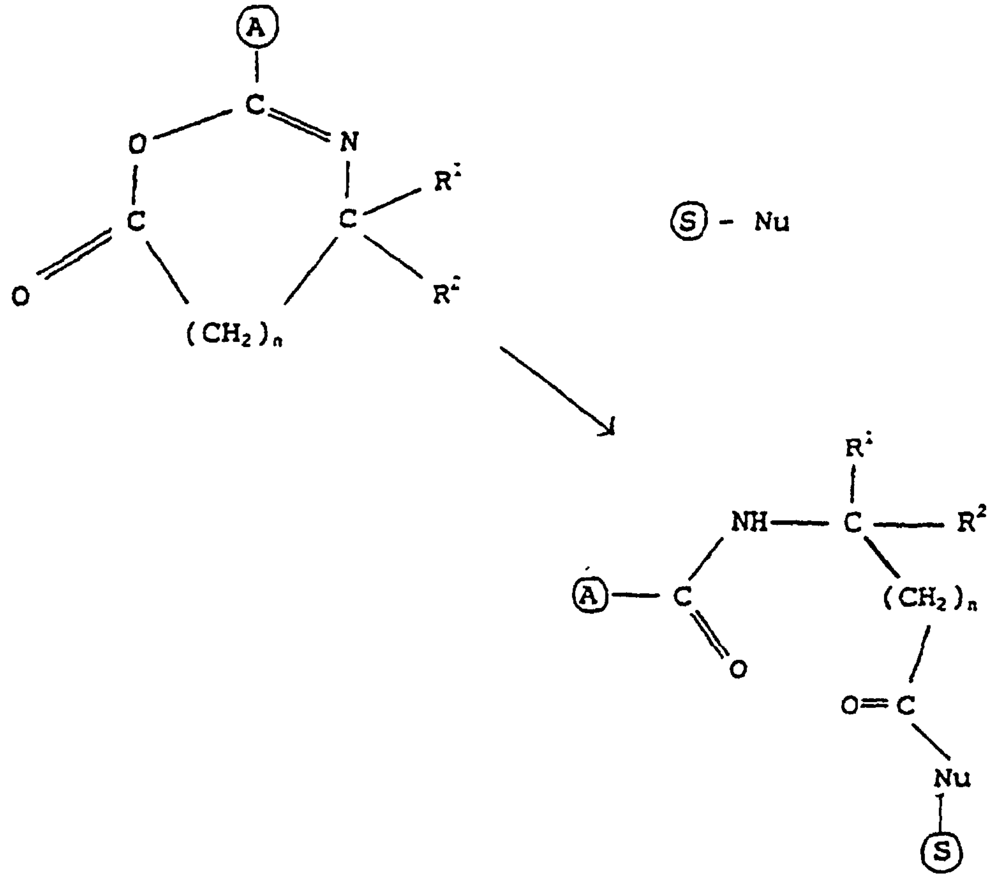

wherein A represents the oral active moiety to which is bonded the azlactone group, S represents the oral surface substrate material and Nu represents a nucleophilic group at the target oral surface.

By this reaction the oral active moiety carried by the azlactone functional group becomes chemically bonded to the oral substrate material via the surface nucleophile, thereby creating a chemical link between the two. This chemisorption creates a bond between oral substrate and oral active moiety, such that the benefit(s) provided by the active agent are relatively long-term and may even be substantially permanent. This is very different from conventional oral treatments, which rely on physisorbtion of the oral active which is to impart its characteristic benefit(s). Because of the much weaker attractive and retentive forces between oral substrate and active agent in conventional systems, these known methods produce only short-lived and distinctly temporary beneficial effects.

The oral active moiety which is azlactone-functionalised to produce the oral treatment agent material in accordance with the invention may for example be a polymer. Various such polymeric materials are suitable, depending upon the particular oral benefit or benefits it is desired to deliver. Preferred polymeric treatment agents include those known polymeric materials for use in conventional cosmetic personal products or dental treatment products for the purpose of film-forming, examples of which include the following:

(i) Silicone polymers and copolymers, such as siloxane gums and resins, non-volatile silicone oils, amino-(or other) functional silicones, end-functionalised silicone polymers and other silicon-containing polymers known for various film-forming applications.
(ii) Aliphatic or aromative hydrocarbon molecules or polymers.
(iii) Perfluoro-aliphatic or -aromatic compounds.
(iv) Modified carbohydrate polymers, e.g. chitosan, Riosan (trade name).
(v) Proteins, such as gelatin.
(vi) Other known film-forming polymers, such as polyvinylpyrrolidone/vinyl acetate copolymers.

Other oral active moieties which may be functionalised with one or more azlactone groups, in accordance with the invention, include:

(vii) Anti-caries agents, e.g. amine fluorides, monosodiumfluorophosphate, casein, plaque buffers such as urea, calcium lactate, calcium glycerophosphate.
(viii) Anti-plaque agents.
(ix) Agents for alleviating sensitive teeth, e.g. Potassium and strontium salts particularly, those of carboxylic acids, materials that form films and block pores.
(x) Pharmaceutical actives for treating disease or other medical condition associated with the oral region, examples of which are many and widespread in the pharmaceutical art, e.g. buprofen, flurbiprofen, aspirin, indomethacin.
(xi) Functional biomolecules, such as peptides, antibodies, enzymes.
(xii) Anti-tartar agents.
(xiii) Agents for treating or preventing bad breath halitosis), e.g. zinc salts.
(xiv) Anti-bacterial agents, e.g. triclosan, chlorohexidine, copper-, zinc- and stannoussalts such as zinc citrate, sodium zinc citrate and stannous pyrophosphate, sanguinarine extract, metronidazole. Further examples of anti-bacterial agents include quaternary ammonium compounds such as chlorhexidine digluconate, hexetidine, octenidine, alexidine; halogenated bisphenolic compounds such as 2,2'methylenebis-(4-chloro-6-bromophenol).
(xv) Anti-calculus agents, e.g. alkalimetal pyrophosphates, hypophosphite-containing polymers, organic phosphonates, phosphocitrates.

The azlactone-functionalised oral treatment species may be prepared by conventional synthetic routes, as will be well known to persons skilled in the chemical art, from (a) at least one reactive species comprising the moiety providing the desired oral treatment benefit, and (b) at least one reactive species comprising the above-defined azlactone group. Suitable reagents, reaction protocols and methods are well described in the chemical and patent literature, and will be readily apparent and available to persons skilled in the art.

One particularly preferred family of reactive species (a) are those containing a vinyl unsaturation, for example a methacryloyl-substituted polymer such as a siloxane, e.g. polydimethylsiloxane. Such monomers are particularly suitable for reaction with a vinyl-unsaturated azlactone as species (b) of the reactive entities and are particularly suitable for radical addition polymerisation using conventional techniques. However, other polymerisation reactions, or other types of reaction, may be used, as are known to and will be well understood by persons skilled in the art and described for example in EP-A-0392735, the disclosure of which is incorporated herein by reference.

The reaction of the azlactone-containing species and the oral treatment active-containing species may take place between one or more of each species, to produce a product for use in the invention which comprises at least one moiety providing the oral treatment benefit and at least one moiety constituting an azlactone "handle" for reaction with the oral substrate surface to be treated.

The reaction may be a copolymerisation reaction involving both species (a) and (b) or may be a terpolymerisation reaction with a third monomer which may be used to control certain properties of the resulting material, for example its reactivity with the nucleophilic substrate surface, its solubility or dispersibility in a given reaction or delivery medium or its action to produce a particular degree of or combination of one or more desired oral treatment benefits. Typical copolymerisation and terpolymerisation reaction protocols are well known in the art and specific examples are given in the Examples hereinbelow. The reaction may alternatively be in the form of a grafting reaction, in which one or more (b) species are grafted onto a backbone of a polymer of species (a), or one or more (a) species are grafted onto a backbone of a polymer of species (b), to give the required azlactone-functionalised oral treatment active product.

Starting materials for the above reactions or polymerisations may be either readily available commercially or may be prepared using known methods and protocols described in the technical and patent literature.

The azlactone-functionalised oral treatment agent used in the invention may be monofunctionalised, i.e. the oral active moiety carries a single azlactone group for reaction with the nucleophilic oral substrate surface, or it may be bis- or multi-functionalised, i.e. the active moiety may carry two or more azlactone functionalities. The latter may be useful for example in achieving a greater degree of chemical bonding of the active agent to the oral substrate or may be useful for generating chemical bonds between adjacent nucleophilic features of a single section of oral substrate.

Depending upon the degree of polymerisation and/or the number and nature of reactive species used, it is possible to produce an azlactone-functionalised oral treatment material having appropriate properties as regards stability in a composition into which it is to be incorporated, reactivity with an intended nucleophilic oral substrate and also solubility in aqueous or non-aqueous media. The latter property may be important in determining the ease with which the active material is incorporated into a desired oral treatment composition and also the efficiency of the means by which it is delivered to the site of reaction on the target oral surface.

Accordingly, in preferred embodiments of the invention, the azlactone-functionalised oral active agent is a

polymeric molecule comprising, in addition to the oral active and azlactone moieties, one or more additional monomer residue units which impart a desired degree of water-solubility to the molecule, by virtue of either or both of the chemical nature of and the proportion in the molecule of such additional monomer residues.

Preferred such terpolymerisation species include for example:-

- polymerizable carboxylates, e.g acrylic or methacrylic acids, esters and salts thereof;
- ethylenically unsaturated acids, e.g. maleic acid, itaconic acid, and esters and salts thereof;
- N-alkyl amine vinyl carboxylates, e.g. N,N-dimethyl ethylamine methacrylate;
- vinyl-containing acids, alcohols, esters and heterocyclic species, e.g. vinylbenzoic acid, vinylalcohol, vinylacetate, vinylpyrrolidone, vinylimidazole, dimethyldiallyammonium chloride (DMDAC).

In certain embodiments of the invention, preferred azlactone-functionalised active materials may be those which are water-soluble or soluble in water/alcohol, e.g. ethanol, mixtures. This may enable compositions according to the present invention to be prepared as aqueous or aqueous/alcoholic pastes, creams, solutions or emulsions, with or without additional ingredients such as those normally found in conventional oral products. The active materials used herein may be manufactured so as to have controlled selected solubility and/or dispersibility properties, thus making them tailor-made for stable incorporation into a particular product formulation and/or type from which they are intended to be delivered. Alternatively, the active materials for use in the invention may be soluble/dispersible in organic solvents only, e.g. alcohols, hydrocarbons, etc.

Water-insoluble or water/alcohol-insoluble azlactone-functionalised active materials may also be preferred in other embodiments of the invention. In such embodiments, and possibly also others, suitable solvents for the azlactone-functionalised oral treatment materials may include organic solvents, such as those well known in the art, e.g. volatile or non-volatile silicones, hydrocarbons, etc. Such solvents may be employed for example for the purpose of pre-dissolving a particular azlactone-functionalised material before incorporation into the final oral composition, which may be aqueous- or non-aqueous-based.

Generally, it is important that any additional ingredients present in the oral compositions of the invention do not affect the stability of the aziactone-functionalised active agent or interfere with its ability to react with a nucleophilic group on the oral substrate surface to which the composition is applied. In this context, whilst it might have been expected that reaction of the functionalised active agent with a nucleophilic group of the substrate competes with hydrolysis of the azlactone moiety in an aqueous reaction medium, it has in fact been found that the reaction of the azlactone group with the substrate nucleophile is favoured over its hydrolysis. This means that it is all the more useful to be able to incorporate the active materials of the present invention in aqueous or aqueous-based products, which are by nature relatively cheap and easy to manufacture, store, transport and use.

<u>Oral compositions comprising the azlactone-functionalised oral treatment agent</u>

In this aspect of the present invention, an oral composition comprises at least one of the above described azlactone-functionalised oral treatment agents, together with any additional ingredients which are normally to be found in oral treatment compositions. One or more of the azlactone-functionalised oral active agents may be used, the use of two or more being possible for example where a combination of benefits is wanted, each derivable from a different active species.

The one or more azlactone-functionalised oral treatment agents may be present in the oral compositions of the invention in comparable or preferably smaller amounts than they would be used in conventional oral treatment compositions relying for their efficacy on physisorption of the active on the substrate. Smaller amounts may be possible than have hitherto been the case because of the tendency for more of the material to be retained long term on the substrate by chemical reaction as opposed to mere physisorption as in the prior art.

Typically, the azlactone-functionalised oral treatment agent is present in the compositions of the invention in an amount from about 0.00001 to 20% by weight of the composition, more preferably from about 0.001 to 15% by weight, even more preferably from about 0.005 to 12% by weight, especially from about 0.01 to 10% by weight. Suitable ranges of amounts may generally depend upon the active in question: for example, azlactone-functionalised siloxane anti-stain agents may be present generally from about 0.01 to 10% by weight of the composition.

An oral composition of the invention may contain one or more surfactants in order to provide detergent action simultaneously with the imparting of the cosmetic, pharmaceutical or therapeutic benefit(s) characteristic of the active agent, and/or possibly to enhance the ease of reaction of the azlactone functional group with the nucleophilic group on the oral substrate.

Preferred surfactants, which may be used singly or in combination, are selected from anionic, cationic, nonionic, amphoteric and zwitterionic surfactants, and mixtures thereof.

Suitable anionic surfactants may include the alkyl sulphates, alkyl ether sulphates, alkaryl sulphonates, alkanoyl isethionates, alkyl succinates, alkyl sulphosuccinates, N-alkoyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl and acyl groups generally contain from 8 to 18 carbon atoms and may be unsaturated . The alkyl ether sulphates, alkyl ether phosphates and alkyl ether carboxylates may contain from 1 to 10 ethylene oxide or propylene oxide units per molecule, and preferably contain 2 to 3 ethylene oxide units per molecule.

Nonionic surfactants suitable for use in compositions of the invention may include condensation products of aliphatic ($C_8$ - $C_{18}$) linear or branched chain alcohols or phenols with alkylene oxides, usually ethylene oxide and generally having from 6 to 30 ethylene oxide groups.

Other suitable nonionics may include mono- or di- alkyl alkanolamides and alkyl polyglycosides (APG's).

Amphoteric and zwitterionic surfactants suitable for use in compositions of the invention may include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms.

Specific examples of all the abovementioned classes of surfactant are well known and well described in the chemical and patent literature.

The surfactant(s) may be present in the oral compositions of the invention in a total amount of from about 0.05 to about 30% by weight, preferably from about 0.1 to about 15 or 20% by weight.

The oral composition of the invention may further comprise any number of stably suspended or dissolved additional ingredients such as are normally included in conventional oral compositions.

Such additional ingredients include for example:

1. sequestering agents;
2. abrasives and polishing agents, e.g. silica, clays, chalk, alumina, calcium phosphates, titanium oxide, magnesium oxide;
3. colouring or dyeing agents, bleaching agents, whitening agents;
4. flavourings, sweeteners;
5. pH adjusting agents, buffers;
6. preservatives, antimicrobial agents;
7. proteins, vitamins, nutrients, stimulants, astringents, anti-radical agents, enzymes, peptides, antibodies;
8. herb or plant extracts, essential oils;
9. suspending agents, emulsifiers, humectants;
10. foam controlling agents;
11. thickening agents, stabilizing agents, gelling agents, natural or synthetic gums and the like;
12. fluoride-containing compounds;
13. effervesing systems, e.g. sodium bicarbonate/citric acid;
14. colour change systems;
15. opacifying agents;
16. metal salts, e.g. zinc, stannous, potassium, strontium;
17. phosphate and polyphosphates;
18. solvents or dispersion media, e.g. water, alcohol (e.g. ethanol, isopropyl alcohol).

Specific examples of all of the abovementioned additional ingredients are well known from the chemical and patent literature, as will be appreciated by persons skilled in the art. Such additional ingredients may be present in oral compositions in conventional amounts, also as are well known and described in the patent and technical literature, e. g. as described in US 4687663, the disclosure of which is incorporated herein by reference.

If desired or as necessary, the oral compositions of the invention may include a catalyst or promoter to initiate or catalyse the reaction of the azlactone group of the active with the nucleophile of the oral substrate.

The oral compositions according to the invention may be provided in any suitable physical form, for example as toothpaste, tooth powders, creams, ointments, mouthwashes, the nature and typical composition of which are well known in the art.

It is generally important that when a given oral substrate surface is treated with a composition of the invention, the physical environment in the composition permits contact of the azlactone-functionalised oral treatment agent with nucleophilic sites on the substrate. Thus, not only is it desirable that the physical structure of the composition allows maximum contact of the active agent with the surface of the substrate, for which reason liquid or paste-like products are preferred, but it is also especially desirable that treatment regimes allow for a period of time during which the active

molecules in the composition remain in contact with the substrate to allow them to react and become bonded thereto.

Accordingly, in preferred methods of the invention wherein the azlactone-functionalised oral treatment agent is applied to the oral substrate surface to impart thereto one or more oral benefits, it is particularly preferable for the treatment to include a retention step during which the composition containing the active is left in contact with the surface for a period of time sufficient for the azlactone rings to react with the nucleophilic sites on the substrate surface.

The invention will now be further illustrated by way of the following non-limiting examples:

EXAMPLES

In the following Examples, the following polymers were used:

Polymer I

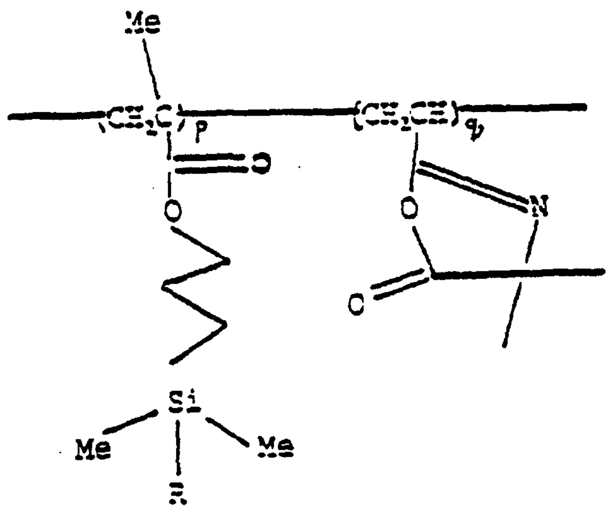

wherein R =

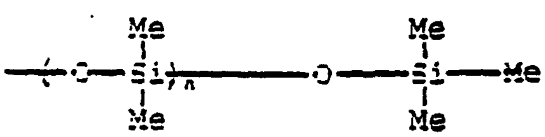

p = 1 to 5, q = 1 to 50, n = 1 to 150.

In preferred examples of Polymer I, p, q and n may take the following values:-

(i) p = 1, q = 3, n = 10;
(ii) p = 1, q = 15, n = 60;
(iii) p = 1, q = 30, n = 130.

Polymer II

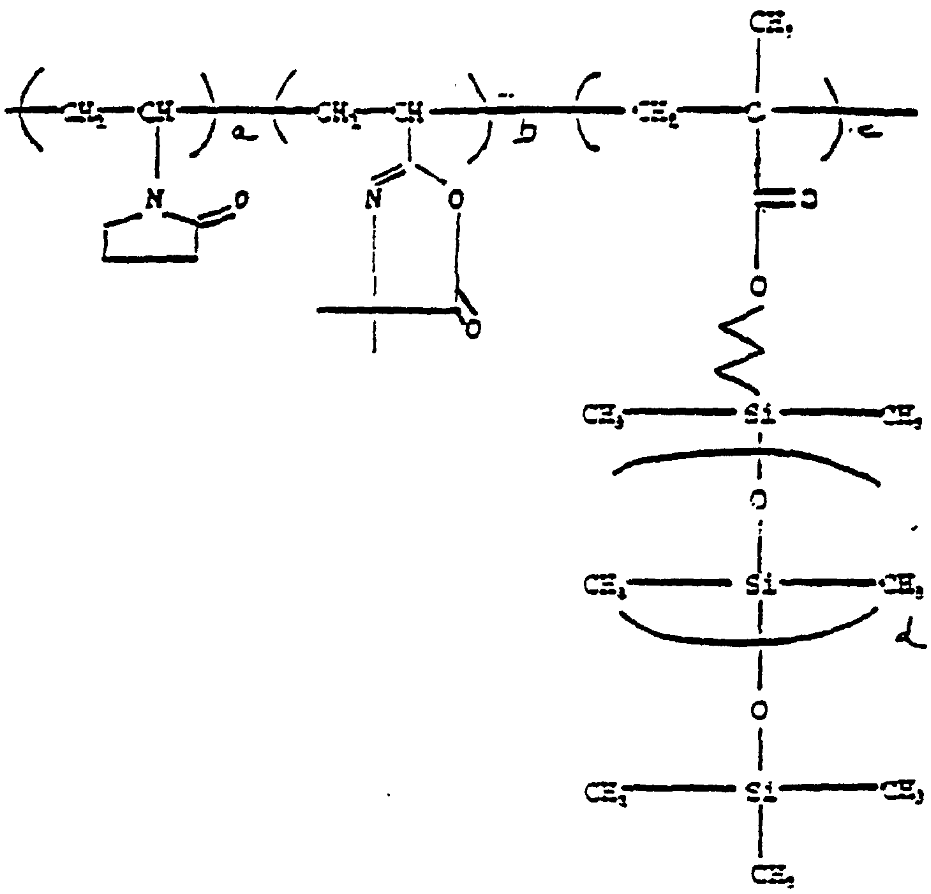

wherein:

a = 1 to 99, preferably 30 to 60;
b = 0.1 to 40, preferably 2 to 20;
c = 1 to 99, preferably 30 to 60;
d = 1 to 150, preferably 5 to 20.

In a preferred example of Polymer II, a, b, c and d take the following values:-

a = 45, b = 10, c = 45, d = 10.
(Note: the values of p, q, a, b and c represent the mole ratios of the monomers used in the reaction for the preparation of the polymers, and do not denote the polymer structure, i.e. whether random or block copolymers.)

The above polymers were prepared by the following methods:

Polymer I

The reactants used were as follows:

(a) 2-Vinyl-4,4-dimethylazlactone (vinylazlactone) supplied by SNPE, Inc., Princeton, NJ.;
(b) Methacryloyl polydimethylsiloxane (PDMS) obtained from Chisso Corporation (Silicone Chemicals Group) Tokyo, Japan.

The silicone chain length of the PDMS starting material was selected so as to give the required value of n in the above formula of the Polymer I product.

Vinylazlactone (3.5g), PDMS (8.5g), AIBN (240mg) and toluene (20ml) were charged into a polymerisation vessel fitted with a nitrogen inlet and a reflux condenser. The polymerisation was carried out at 70°C for 48 hours. The resulting viscous solution was precipitated by dropwise addition into excess methanol with vigorous stirring. The polymer was vacuum dried at 50°C for four hours and characterised by NMR and FT-IR spectroscopy.

Polymer II

Vinylazlactone (0.5g), PDMS (11.25g), vinylpyrrolidone (5.11g), AIBN (123mg) and toluene (60ml) were charged into a polymerisation vessel, and the polymerisation reaction was conducted as above and purified and characterised in a similar manner.

Examples 1 (Polymer I) and 2 (Polymer II)

To imitate the natural pellicle present on dental surfaces, samples of substrate were prepared by taking hydroxyapatite discs and immersing them in sterile saliva at 37°C overnight.

The colour of the discs was then measured using a Minolta chromameter in the L*a*b* mode.

A solution A of Polymer I wherein in its formula p = 1, q = 3, n = 10 was made up in isopropyl alcohol, at 5% w/w concentration. A similar 5% w/w solution B of Polymer II (wherein in its formula a - 45, b = 10, c = 45, d = 10) was made up in distilled water. Ten prepared discs were immersed in each of the solutions for 5 minutes and then removed and rinsed with water.

To assess the anti-staining efficacy of the polymers, each group of ten treated discs were then immersed in an aqueous stain broth made up of a mixture of tea, coffee, ferrous ammonium sulphate and saliva for several days. As time progressed, the amount of stain build-up was measured using the chromameter using the L value (i.e. level of whiteness).

As controls, ten prepared discs were also immersed for the same periods of time in the respective pure solvents, without any polymer, and corresponding whiteness measurements taken.

The results are shown in Tables 1 and 2 below. The $\Delta L^*$ value is defined as $\Delta L^* = L_o^* - L_t^*$, where $L_o^*$ is the measured L* whiteness value of the unstained discs measured at time zero, and $L^*_t$ is the measured L whiteness value of the stained discs at time t (days). Each $\Delta L^*$ value is the average for the ten discs of each group, i.e. $\Delta L^* = \frac{\Sigma L^*}{n}$, wherein n = 10.

Table 1

| | ΔL* | |
|---|---|---|
| Time (days) | Solution A (5% Polymer I in IPA) | IPA 100% (control) |
| 0 | 0 | 0 |
| 1 | 12 | 16 |
| 3 | 16 | 23 |
| 6 | 17 | 25.5 |
| 7 | 17 | 26.5 |

Table 2

| | ΔL* | |
|---|---|---|
| Time (days) | Solution B (5% Polymer II in water) | Water 100% (control) |
| 0 | 0 | 0 |
| 1 | 3 | 7 |
| 3 | 4 | 13 |
| 6 | 5 | 16 |

The results clearly show that discs pretreated with Polymers I and II exhibited markedly reduced staining

compared with the control discs which were untreated with polymer.

Example 3 (Polymer II)

The experimental test procedure of Examples 1 and 2 above was repeated using a 5% w/w solution C of the same Polymer II species, but this time in ethanol as the solvent. The pure ethanol was used for the control disc samples untreated with polymer.

The results of the whiteness value measurements are shown in Table 3 below. (The figure in brackets after each ΔL* value is the standard deviation.)

Table 3

| | ΔL* | |
|---|---|---|
| Time (days) | Solution C (5% Polymer II in ethanol) | Ethanol 100% (control) |
| 0 | 0 | 0 |
| 1 | 4.9 (0.9) | 22.4 (1.9) |
| 4 | 7.4 (1.3) | 29.8 (2.2) |
| 6 | 8.8 (1.8) | 31.8 (2.4) |

Example 4 (Polymer II, varying concentrations)

A further series of experiments was conducted, using the same test procedures of Examples 1 to 3 above, in which solutions (solutions D, E and F) of varying concentrations of the same Polymer II species in ethanol were tested for their anti-stain properties as a function of polymer concentration.

The mean results of the whiteness value measurements are shown in Table 4 below. (The figure in brackets after each mean ΔL* value is the standard deviation.)

Table 4

| | Mean ΔL* | | | |
|---|---|---|---|---|
| Solution tested | Time (days) | | | |
| | 0 | 1 | 2 | 5 |
| D (5% w/w Polymer II in ethanol) | 0 | 10.7 (0.9) | 16.5 (1.7) | 19.8 (2.3) |
| E (3% w/w Polymer II in ethanol) | 0 | 26.2 (3.6) | 26.1 (2.6) | 30.8 (3.0) |
| F (1% w/w Polymer II | 0 | 25.6 (2.8) | 25.7 (2.1) | 30.7 (3.2) |
| Ethanol 100% (control) | 0 | 28.9 (3.0) | 28.8 (2.2) | 35.3 (2.0) |

## Claims

**1.** A method of cosmetic oral treatment wherein a cosmetic oral treatment agent is applied to an oral surface, the method comprising:

(a) providing the cosmetic oral treatment agent as molecules thereof having chemically bonded thereto at least one azlactone substituent moiety for reaction with a nucleophilic reactive site on the oral surface to be treated; and

(b) applying the azlactone-functionalised cosmetic oral treatment agent to the oral surface to effect reaction of the or each azlactone moiety with a or a respective nucleophilic site on the surface;

wherein the molecules of the cosmetic oral treatment agent are chemically bound to the surface to impart thereto one or more oral benefits characteristic of said oral treatment agent.

**2.** A method according to claim 1, wherein the nucleophilic reactive site on the oral substrate surface comprises a nitrogen-containing, oxygen-containing or sulphur-containing functional group.

**3.** A method according to claim 1, wherein the nucleophilic reactive site is a nitrogen-containing functional group and the reaction is effectable in a substantially aqueous medium.

**4.** A method according to claim 1, wherein the nucleophilic reactive site comprises an oxygen-containing functional group and the reaction is effectable in a substantially non-aqueous medium.

**5.** A method according to any one of claims 1 to 4, wherein the oral surface is selected from teeth and gums.

**6.** A method according to any one of claims 1 to 4, wherein the oral surface is pellicle on the surface of teeth.

**7.** A method according to any one of claims 1 to 6, wherein the or each azlactone substituent moiety is selected from groups defined by the following formula:

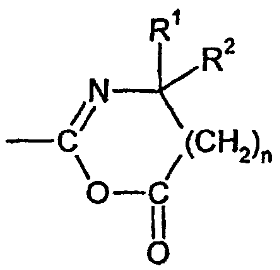

wherein:

$R^1$ and $R^2$ are the same or different and each is independently selected from H, an alkyl group having from 1 to 14 carbon atoms, particularly lower ($C_1$-$C_4$) alkyl e.g. methyl, a cycloalkyl group having from 3 to 14 carbon atoms, an aryl group having from 5 to 12 ring carbon atoms, an arenyl group having from 6 to 26 carbon atoms and from 0 to 3 S, N or O heteroatoms, or $R^1$ and $R^2$ taken together with the carbon atom to which they are joined can form a carbocyclic ring containing 4 to 12 ring atoms; and,
n is an integer of from 0 to 12.

**8.** A method according to any one of claims 1 to 7, wherein the oral active moiety of the said oral treatment agent is a polymeric material.

**9.** A method according to any one of claims 1 to 7, wherein the oral treatment agent is an anti-stain agent, an anti-malodour agent, or a teeth whitening agent.

**10.** A method according to any one of claims 1 to 8, wherein the oral treatment agent is an azlactone-functionalised anti-stain agent.

**11.** A method according to claim 9, wherein the oral treatment agent is an azlactone-functionalised polysiloxane.

**12.** An oral treatment composition in the form of a mouthwash, toothpaste, or tooth powder for treating an oral surface to impart thereto one or more cosmetic, pharmaceutical or therapeutic benefits, the composition comprising dissolved or dispersed particles of an oral treatment agent having chemically bonded thereto at least one azlactone substituent moiety capable of reaction with a nucleophilic reactive site on the oral surface to be treated.

**13.** A composition according to claim 12, wherein the nucleophilic reactive site on the oral substrate surface comprises a nitrogen-containing, oxygen-containing or sulphur-containing functional group.

**14.** A composition according to claim 12, wherein the nucleophilic reactive site is a nitrogen-containing functional group and the reaction is effectable in a substantially aqueous medium.

**15.** A composition according to claim 12, wherein the nucleophilic reactive site comprises an oxygen-containing functional group and the reaction is effectable in a substantially non-aqueous medium.

**16.** A composition according to any one of claims 12 to 15, wherein the oral surface is selected from teeth and gums.

**17.** A composition according to any one of claims 12 to 15, wherein the oral surface is pellicle on the surface of teeth.

**18.** A composition according to any one of claims 12 to 17, wherein the or each azlactone substituent moiety is selected from groups defined by the following formula:

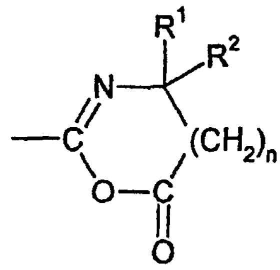

wherein:

$R^1$ and $R^2$ are the same or different and each is independently selected from H, an alkyl group having from 1 to 14 carbon atoms, particularly lower ($C_1$-$C_4$) alkyl e.g. methyl, a cycloalkyl group having from 3 to 14 carbon atoms, an aryl group having from 5 to 12 ring carbon atoms, an arenyl group having from 6 to 26 carbon atoms and from 0 to 3 S, N or O heteroatoms, or $R^1$ and $R^2$ taken together with the carbon atom to which they are joined can form a carbocyclic ring containing 4 to 12 ring atoms; and,
n is an integer of from 0 to 12.

**19.** A composition according to any one of claims 12 to 18, wherein the oral active moiety of the said oral treatment agent is a polymeric material.

**20.** A composition according to any one of claims 12 to 18, wherein the oral treatment agent is an anti-caries agent, an anti-plaque agent, an agent for alleviating sensitive teeth, an anti-calculus agent, or an anti-tartar agent.

**21.** A composition according to any one of claims 12 to 18, wherein the oral treatment agent is an anti-stain agent, an anti-malodour agent, or a teeth whitening agent.

**22.** A composition according to any one of claims 12 to 19, wherein the oral treatment agent is an aziactone-functionalised anti-stain agent.

**23.** A composition according to claim 22, wherein the oral treatment agent is an azlactone-functionalised polysiloxane.

**24.** Use of an azlactone-functionalised oral treatment agent for the manufacture of a medicament for use in the treatment of an oral condition by applying said oral treatment agent to an oral surface to be treated, wherein the oral treatment agent has chemically bonded thereto at least one azlactone substituent moiety capable of reaction with a nucleophilic reactive site on an oral surface to be treated.

**25.** Use according to claim 24, wherein the nucleophilic reactive site on the oral substrate surface comprises a nitrogen-containing, oxygen-containing or sulphur-containing functional group.

**26.** Use according to claim 24, wherein the nucleophilic reactive site is a nitrogen-containing functional group and the reaction is effectable in a substantially aqueous medium.

**27.** Use according to claim 24, wherein the nucleophilic reactive site comprises an oxygen-containing functional group and the reaction is effectable in a substantially non-aqueous medium.

**28.** Use according to any one of claims 24 to 27, wherein the oral surface is selected from teeth and gums.

**29.** Use according to any one of claims 24 to 27, wherein the oral surface is pellicle on the surface of teeth.

**30.** Use according to any one of claims 24 to 29, wherein the or each azlactone substituent moiety is selected from groups defined by the following formula:

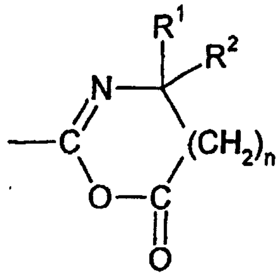

wherein:

$R^1$ and $R^2$ are the same or different and each is independently selected from H, an alkyl group having from 1 to 14 carbon atoms, particularly lower ($C_1$-$C_4$) alkyl e.g. methyl, a cycloalkyl group having from 3 to 14 carbon atoms, an aryl group having from 5 to 12 ring carbon atoms, an arenyl group having from 6 to 26 carbon atoms and from 0 to 3 S, N or O heteroatoms, or $R^1$ and $R^2$ taken together with the carbon atom to which they are joined can form a carbocyclic ring containing 4 to 12 ring atoms; and,
n is an integer of from 0 to 12.

**31.** Use according to any one of claims 24 to 30, wherein the oral active moiety of the said oral treatment agent is a polymeric material.

**32.** Use according to any one of claims 24 to 30, wherein the oral treatment agent is an anti-caries agent, an anti-plaque agent, an agent for alleviating sensitive teeth, an anti-calculus agent, or an anti-tartar agent.

**Patentansprüche**

**1.** Verfahren zur kosmetischen oralen Behandlung, wobei ein kosmetisches orales Behandlungsmittel auf eine orale Oberfläche aufgetragen wird, wobei das Verfahren umfasst:

(a) Bereitstellen des kosmetischen oralen Behandlungsmittels als Moleküle davon, mit mindestens einer daran chemisch gebundenen Azlactonsubstituenteneinheit, zur Reaktion mit einer nukleophilen reaktiven Stelle an der zu behandelnden oralen Oberfläche; und
(b) Auftragen des Azlacton-funktionalisierten kosmetischen oralen Behandlungsmittels auf die orale Oberfläche, um eine Reaktion der oder jeder Azlactoneinheit mit einer oder einer entsprechenden nukleophilen Stelle auf der Oberfläche zu bewirken,

wobei die Moleküle des kosmetischen oralen Behandlungsmittels chemisch an die Oberfläche gebunden werden, um daran einen oder mehrere orale Vorteile zu verleihen, die für das orale Behandlungsmittel charakteristisch sind.

**2.** Verfahren nach Anspruch 1, wobei die nukleophile reaktive Stelle auf der oralen Substratoberfläche eine Stickstoff-enthaltende, Sauerstoff-enthaltende oder Schwefel-enthaltende funktionelle Gruppe umfasst.

**3.** Verfahren nach Anspruch 1, wobei die nukleophile reaktive Stelle eine Stickstoff-enthaltende funktionelle Gruppe ist und die Reaktion in einem im Wesentlichen wässerigen Medium bewirkbar ist.

**4.** Verfahren nach Anspruch 1, wobei die nukleophile reaktive Stelle eine Sauerstoff-enthaltende funktionelle Gruppe umfasst und die Reaktion in einem im Wesentlichen nichtwässerigen Medium bewirkbar ist.

**5.** Verfahren nach einem der Ansprüche 1 bis 4, wobei die orale Oberfläche aus Zähnen und Zahnfleisch ausgewählt ist.

**6.** Verfahren nach einem der Ansprüche 1 bis 4, wobei die orale Oberfläche eine Membran auf der Oberfläche der Zähne ist.

**7.** Verfahren nach einem der Ansprüche 1 bis 6, wobei der oder jede Azlactonsubstituenteneinheit aus Gruppen ausgewählt ist, die durch die nachstehende Formel definiert sind:

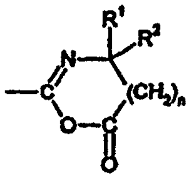

worin

$R^1$ und $R^2$ gleich oder verschieden sind und jeweils unabhängig ausgewählt sind aus H, einer Alkylgruppe mit 1 bis 14 Kohlenstoffatomen, insbesondere Nieder-($C_1$-$C_4$)-alkyl, beispielsweise Methyl, einer Cycloalkylgruppe mit 3 bis 14 Kohlenstoffatomen, einer Arylgruppe mit 5 bis 12 Ringkohlenstoffatomen, einer Arenylgruppe mit 6 bis 26 Kohlenstoffatomen und 0 bis 3 S-, N- oder O-Heteroatomen, oder $R^1$ und $R^2$, zusammengenommen mit dem Kohlenstoffatom, an das sie gebunden sind, einen carbocyclischen Ring, der 4 bis 12 Ringatome enthält, bilden können; und

n eine ganze Zahl von 0 bis 12 ist.

**8.** Verfahren nach einem der Ansprüche 1 bis 7, wobei die orale aktive Einheit des oralen Behandlungsmittels ein polymeres Material ist.

**9.** Verfahren nach einem der Ansprüche 1 bis 7, wobei das orale Behandlungsmittel ein Antiverfärbungsmittel, ein Antimundgeruchmittel oder ein Zahnweißungsmittel ist.

**10.** Verfahren nach einem der Ansprüche 1 bis 8, wobei das orale Behandlungsmittel ein Azlacton-funktionalisiertes Antiverfärbungsmittel ist.

**11.** Verfahren nach Anspruch 9, wobei das orale Behandlungsmittel ein Azlacton-funktionalisiertes Polysiloxan ist.

**12.** Orale Behandlungszusammensetzung in Form einer Mundwäsche, Zahnpaste oder eines Zahnpulvers zum Behandeln einer oralen Oberfläche, um daran einen oder mehrere kosmetische, pharmazeutische oder therapeutische Vorteile zu verleihen, wobei die Zusammensetzung gelöste oder dispergierte Teilchen von einem oralen Behandlungsmittel, mit mindestens einer chemisch daran gebundenen Azlactonsubstituenteneinheit, die mit einer nukleophilen reaktiven Stelle auf der oralen, zu behandelnden Oberfläche reagieren kann, aufweist.

**13.** Zusammensetzung nach Anspruch 12, wobei die nukleophile reaktive Stelle auf der oralen Substratoberfläche eine Stickstoff-enthaltende, Sauerstoff-enthaltende oder Schwefel-enthaltende funktionelle Gruppe umfasst.

**14.** Zusammensetzung nach Anspruch 12, wobei die nukleophile reaktive Stelle eine Stickstoff-enthaltende funktionelle Gruppe darstellt und die Reaktion in einem im Wesentlichen wässerigen Medium bewirkbar ist.

**15.** Zusammensetzung nach Anspruch 12, wobei die nukleophile reaktive Stelle eine Sauerstoff-enthaltende funktionelle Gruppe umfasst und die Reaktion in einem im Wesentlichen nichtwässerigen Medium bewirkbar ist.

**16.** Zusammensetzung nach einem der Ansprüche 12 bis 15, wobei die orale Oberfläche aus Zähnen und Zahnfleisch ausgewählt ist.

**17.** Zusammensetzung nach einem der Ansprüche 12 bis 15, wobei die orale Oberfläche eine Membran auf der Oberfläche der Zähne ist.

**18.** Zusammensetzung nach einem der Ansprüche 12 bis 17, wobei die oder jede Azlactonsubstituenteneinheit aus Gruppen ausgewählt ist, die durch die nachstehende Formel definiert sind:

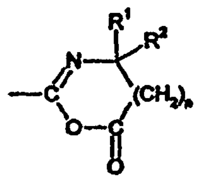

worin

$R^1$ und $R^2$ gleich oder verschieden sind und jeweils unabhängig ausgewählt sind aus H, einer Alkylgruppe mit 1 bis 14 Kohlenstoffatomen, insbesondere Nieder-($C_1$-$C_4$)-alkyl, beispielsweise Methyl, einer Cycloalkylgruppe mit 3 bis 14 Kohlenstoffatomen, einer Arylgruppe mit 5 bis 12 Ringkohlenstoffatomen, einer Arenylgruppe mit 6 bis 26 Kohlenstoffatomen und 0 bis 3 S-, N- oder O-Heteroatomen, oder $R^1$ und $R^2$, zusammengenommen mit dem Kohlenstoffatom, an das sie gebunden sind, einen carbocyclischen Ring, der 4 bis 12 Ringatome enthält, bilden können; und

n eine ganze Zahl von 0 bis 12 ist.

**19.** Zusammensetzung nach einem der Ansprüche 12 bis 18, wobei die orale aktive Einheit des oralen Behandlungsmittels ein polymeres Material ist.

**20.** Zusammensetzung nach einem der Ansprüche 12 bis 18, wobei das orale Behandlungsmittel ein Antikariesmittel, ein Antiplaquemittel, ein Mittel zum Lindern von empfindlichen Zähnen, ein Anticalculusmittel oder ein Antizahnsteinmittel ist.

**21.** Zusammensetzung nach einem der Ansprüche 12 bis 18, wobei das orale Behandlungsmittel ein Antiverfärbungsmittel, ein Antimundgeruchmittel oder ein Zahnweißungsmittel ist.

**22.** Zusammensetzung nach einem der Ansprüche 12 bis 19, wobei das orale Behandlungsmittel ein Azlacton-funktionalisiertes Antiverfärbungsmittel ist.

**23.** Zusammensetzung nach Anspruch 22, wobei das orale Behandlungsmittel ein Azlacton-funktionalisiertes Polysiloxan ist.

**24.** Verwendung eines Azlacton-funktionalisierten oralen Behandlungsmittels zur Herstellung eines Arzneimittels zur Verwendung bei der Behandlung eines oralen Zustands durch Auftragen des oralen Behandlungsmittels auf eine zu behandelnde orale Oberfläche, wobei das orale Behandlungsmittel mindestens eine chemisch daran gebundene Azlactonsubstituenteneinheit, die mit einer nukleophilen reaktiven Stelle auf der oralen, zu behandelnden Oberfläche reagieren kann, aufweist.

**25.** Verwendung nach Anspruch 24, wobei die nukleophile reaktive Stelle auf der oralen Substratoberfläche eine Stickstoff-enthaltende, Sauerstoff-enthaltende oder Schwefel-enthaltende funktionelle Gruppe umfasst.

**26.** Verwendung nach Anspruch 24, wobei die nukleophile reaktive Stelle eine Stickstoff-enthaltende funktionelle Gruppe darstellt und die Reaktion in einem im Wesentlichen wässerigen Medium bewirkbar ist.

**27.** Verwendung nach Anspruch 24, wobei die nukleophile reaktive Stelle eine Sauerstoff-enthaltende funktionelle Gruppe umfasst und die Reaktion in einem im Wesentlichen nichtwässerigen Medium bewirkbar ist.

**28.** Verwendung nach einem der Ansprüche 24 bis 27, wobei die orale Oberfläche aus Zähnen und Zahnfleisch ausgewählt ist.

**29.** Verwendung nach einem der Ansprüche 24 bis 27, wobei die orale Oberfläche eine Membran auf der Oberfläche der Zähne ist.

**30.** Verwendung nach einem der Ansprüche 24 bis 29, wobei der oder jede Azlactonsubstituenteneinheit aus Gruppen

ausgewählt ist, die durch die nachstehende Formel definiert sind:

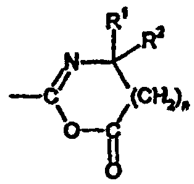

worin

$R^1$ und $R^2$ gleich oder verschieden sind und jeweils unabhängig ausgewählt sind aus H, einer Alkylgruppe mit 1 bis 14 Kohlenstoffatomen, insbesondere Nieder-($C_1$-$C_4$)-alkyl, beispielsweise Methyl, einer Cycloalkylgruppe mit 3 bis 14 Kohlenstoffatomen, einer Arylgruppe mit 5 bis 12 Ringkohlenstoffatomen, einer Arenylgruppe mit 6 bis 26 Kohlenstoffatomen und 0 bis 3 S-, N- oder O-Heteroatomen, oder $R^1$ und $R^2$, zusammengenommen mit dem Kohlenstoffatom, an das sie gebunden sind, einen carbocyclischen Ring, der 4 bis 12 Ringatome enthält, bilden können; und

n eine ganze Zahl von 0 bis 12 ist.

**31.** Verwendung nach einem der Ansprüche 24 bis 30, wobei die orale aktive Einheit des oralen Behandlungsmittels ein polymeres Material ist.

**32.** Verwendung nach einem der Ansprüche 24 bis 30, wobei das orale Behandlungsmittel ein Antikariesmittel, ein Antiplaquemittel, ein Mittel zum Lindern von empfindlichen Zähnen, ein Anticalculusmittel oder ein Antizahnsteinmittel ist.

**Revendications**

**1.** Procédé pour un traitement buccal cosmétique dans lequel un agent de traitement buccal cosmétique est appliqué sur la surface buccale, le procédé comprenant :

(a) fournir l'agent de traitement buccal cosmétique sous forme de molécules de celui-ci, ayant chimiquement fixé dessus au moins un fragment substituant d'azlactone pour la réaction avec un site de nucléophile réactif sur la surface buccale destinée à être traiter ; et

(b) appliquer l'agent de traitement buccal cosmétique rendu fonctionnel par l'azlactone, sur la surface buccale, pour effectuer la réaction du ou de chaque fragment d'azlactone, avec l'un des ou un site nucléophile respectif sur la surface ;

dans lequel les molécules de l'agent de traitement buccal cosmétique sont chimiquement fixées à la surface, pour lui transmettre un ou plusieurs bénéfices buccaux caractéristiques dudit agent de traitement buccal.

**2.** Procédé selon la revendication 1, dans lequel le site réactif nucléophile sur la surface du substrat buccal comprend un groupement fonctionnel contenant un azote, un oxygène ou un soufre.

**3.** Procédé selon la revendication 1, dans lequel le site réactif nucléophile est un groupement fonctionnel contenant un azote et la réaction est réalisable dans un milieu substantiellement aqueux.

**4.** Procédé selon la revendication 1, dans lequel le site réactif nucléophile contient un groupement fonctionnel contenant un azote, et la réaction est réalisable dans un milieu substantiellement aqueux.

**5.** Procédé selon l'une quelconque des revendications 1 à 4, dans lequel la surface buccale est choisie entre les dents et les gencives.

**6.** Procédé selon l'une quelconque des revendications 1 à 4, dans lequel la surface buccale est la pellicule sur la surface des dents.

**7.** Procédé selon l'une quelconque des revendications 1 à 6, dans lequel le ou chaque fragment substituant d'azlactone est choisi parmi les groupes définis par la formule suivante :

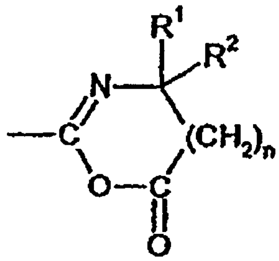

dans lequel :

$R^1$ et $R^2$ sont identiques ou différents, et chacun est indépendamment choisi parmi H, un groupement alkyle ayant 1 à 14 atomes de carbone, en particulier les alkyles inférieurs ($C_1$-$C_4$) par exemple le méthyle, un groupement cycloalkyle ayant 3 à 14 atomes de carbone, un groupement aryle ayant 5 à 12 atomes de carbone dans le cycle, un groupement arényle ayant 6 à 26 atomes de carbone et de 0 à 3 hétéroatomes S, N ou O, ou $R^1$ et $R^2$ pris ensembles avec l'atome de carbone auquel ils sont liés, peuvent former un cycle carbocyclique contenant 4 à 12 atomes dans le cycle ; et,
n est un nombre entier de 0 à 12.

**8.** Procédé selon l'une quelconque des revendications 1 à 7, dans lequel le fragment actif buccal dudit agent de traitement buccal est un matériau polymérique.

**9.** Procédé selon l'une quelconque des revendications 1 à 7, dans lequel l'agent de traitement buccal est un agent anti-colorant, un agent anti-mauvaise odeur ou un agent blanchissant les dents.

**10.** Procédé selon l'une quelconque des revendications 1 à 8, dans lequel l'agent de traitement buccal est un agent anti-colorant rendu fonctionnel par l'azlactone.

**11.** Procédé selon la revendication 9, dans laquelle l'agent de traitement buccal est un polysiloxane rendu fonctionnel par l'azlactone.

**12.** Composition de traitement buccal sous la forme d'un bain de bouche, d'une pâte dentifrice ou d'une poudre dentaire pour traiter une surface buccale pour transmettra à celle-ci un ou plusieurs bénéfices cosmétiques, pharmaceutiques ou thérapeutiques, la composition comprenant des particules dissoutes ou dispersées d'un agent de traitement buccal ayant chimiquement fixé dessus au moins un fragment substituant d'azlactone capable d'une réaction avec un site réactif nucléophile sur la surface buccale à traiter.

**13.** Composition selon la revendication 12, dans laquelle le site réactif nucléophile sur la surface des substrats buccaux comprend un groupement fonctionnel contenant un azote, un oxygène ou un soufre.

**14.** Composition selon la revendication 12, dans laquelle le site réactif nucléophile est un groupement fonctionnel contenant un azote, et la réaction est réalisable dans un milieu substantiellement aqueux.

**15.** Composition selon la revendication 12, dans laquelle le site réactif nucléophile comprend un groupement fonctionnel contenant un oxygène, et la réaction est réalisable dans un milieu substantiellement aqueux.

**16.** Composition selon l'une quelconque des revendications 12 à 15, dans laquelle la surface buccale est choisie entre les dents et les gencives.

**17.** Composition selon l'une quelconque des revendications 12 à 15, dans laquelle la surface buccale est la pellicule sur la surface des dents.

**18.** Composition selon l'une quelconque des revendications 12 à 17, dans laquelle le ou chaque fragment substituant d'azlactone est choisi parmi les groupes définis par la formule suivante :

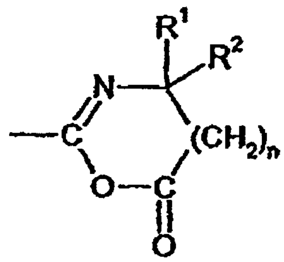

dans lequel :

$R^1$ et $R^2$ sont identiques ou différents et chacun est indépendamment choisi parmi H, un groupement alkyle ayant 1 à 14 atomes de carbone, en particulier les alkyles inférieurs ($C_1$-$C_4$) par exemple le méthyle, un groupement cycloalkyle ayant 3 à 14 atomes de carbone, un groupement aryle ayant 5 à 12 atomes de carbone dans le cycle, un groupement arényle ayant 6 à 26 atomes de carbone et de 0 à 3 hétéroatomes S, N ou O ou $R^1$ et $R^2$ pris ensembles avec l'atome de carbone auquel ils sont liés peuvent former un cycle carbocyclique contenant 4 à 12 atomes dans le cycle ; et,
n est un nombre entier de 0 à 12.

**19.** Composition selon l'une quelconque des revendications 12 à 18, dans laquelle le fragment actif buccal dudit agent de traitement buccal est un matériau polymérique.

**20.** Composition selon l'une quelconque des revendications 12 à 18, dans laquelle l'agent de traitement buccal est un agent anti-caries, un agent anti-plaque, un agent pour soulager les dents sensibles, un agent anti-calculs ou un agent anti-tartre.

**21.** Composition selon l'une quelconque des revendications 12 à 18, dans laquelle l'agent de traitement buccal est un agent anti-colorant, un agent anti-mauvaise odeur ou un agent blanchissant les dents.

**22.** Composition selon l'une quelconque des revendications 12 à 19, dans laquelle l'agent de traitement buccal est un agent anti-colorant rendu fonctionnel par l'azlactone.

**23.** Composition selon la revendication 22, dans laquelle l'agent de traitement buccal est un polysiloxane rendu fonctionnel par l'azlactone.

**24.** Utilisation d'un agent de traitement buccal rendu fonctionnel par l'azlactone pour la fabrication d'un médicament pour une utilisation dans le traitement d'une affection buccale en appliquant ledit traitement buccal sur une surface buccale à traiter, dans laquelle l'agent de traitement buccal a chimiquement fixé dessus au moins un fragment substituant d'azlactone capable d'une réaction avec un site réactif nucléophile sur une surface buccale à traiter.

**25.** Utilisation selon la revendication 24, dans laquelle le site réactif nucléophile sur la surface du substrat buccal comprend un groupement fonctionnel contenant un azote, un oxygène ou un soufre.

**26.** Utilisation selon la revendication 24, dans laquelle le site réactif nucléophile est un groupement fonctionnel contenant un azote, et la réaction est réalisable dans un milieu substantiellement aqueux.

**27.** Utilisation selon la revendication 24, dans laquelle le site réactif nucléophile comprend un groupement fonctionnel contenant de l'oxygène, et la réaction est réalisable dans un milieu substantiellement aqueux.

**28.** Utilisation selon l'une quelconque des revendications 24 à 27, dans laquelle la surface buccale est choisie entre les dents et les gencives.

**29.** Utilisation selon l'une quelconque des revendications 24 à 27, dans laquelle la surface buccale est la pellicule sur la surface des dents.

**30.** Utilisation selon l'une quelconque des revendications 24 à 29, dans laquelle le ou chaque fragment substituant

d'azlactone est choisi parmi les groupes définis par la formule suivante :

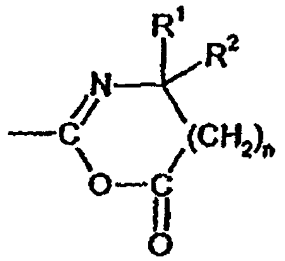

dans lequel :

$R^1$ et $R^2$ sont identiques ou différents et chacun est indépendamment choisi parmi H, un groupement alkyle ayant 1 à 14 atomes de carbone, en particulier les alkyles inférieurs ($C_1$-$C_4$) par exemple le méthyle, un groupement cycloalkyle ayant 3 à 14 atomes de carbone, un groupement aryle ayant 5 à 12 atomes de carbone dans le cycle, un groupement arényle ayant 6 à 26 atomes de carbone et de 0 à 3 hétéroatomes S, N ou O, ou $R^1$ et $R^2$ pris ensembles avec l'atome de carbone auquel ils sont liés peuvent former un cycle carbocyclique contenant 4 à 12 atomes dans le cycle ; et,
n est un nombre entier de 0 à 12.

**31.** Utilisation selon l'une quelconque des revendications 24 à 30, dans laquelle le fragment actif buccal dudit agent de traitement buccal est un matériau polymérique.

**32.** Utilisation selon l'une quelconque des revendications 24 à 30, dans laquelle l'agent de traitement buccal est un agent anti-caries, un agent anti-plaque, un agent pour soulager les dents sensibles, un agent anti-calculs ou un agent anti-tartre.